# EUROPÄISCHE PATENTSCHRIFT

(11) **EP 4 203 817 B1**
(45) Veröffentlichungstag und Bekanntmachung des Hinweises auf die Patenterteilung: **30.04.2025**
(21) Anmeldenummer: 21766439.0
(22) Anmeldetag: 19.08.2021
(51) Int. Cl.: A61B 17/32

(54) **CHIRURGISCHER SHAVER**
SURGICAL SHAVER
RASOIR CHIRURGICAL

(30) Priorität: 31.08.2020 DE 102020122718
(43) Veröffentlichungstag der Anmeldung: 05.07.2023
(73) Patentinhaber: Behrendt, Sven, 44263 Dortmund (DE)
(72) Erfinder: Behrendt, Sven, 44263 Dortmund (DE)
(74) Vertreter: Meinke, Jochen
(86) Internationale Anmeldenummer: PCT/EP2021/073065
(87) Internationale Veröffentlichungsnummer: WO 2022/043189

(56) Entgegenhaltungen:
- DE-A1- 102019 105 514
- US-A- 4 834 729
- US-A1- 2008 249 553
- US-B1- 6 419 684

## Beschreibung

Die Erfindung betrifft einen chirurgischen Shaver mit einer um ihre Längsachse drehbaren rohrförmigen Innenhülse, die mit einem Drehantrieb verbindbar ist, und einer die Innenhülse umgebenden rohrförmigen Außenhülse.

Arthroskopische und endoskopische Verfahren werden häufig zur Diagnose und Behandlung von Gelenkproblemen durchgeführt. So können z.B. Bandrekonstruktionen, Knochenauffüllung und ein Ersatz eines verschlissenen Meniskus mit arthroskopischen Rekonstruktionsverfahren durchgeführt werden, bei denen der Chirurg in der Regel durch eine Reihe relativ kleiner Öffnungen arbeiten muss. Bei solchen Verfahren kann es notwendig werden, z.B. Meniskus- oder anderes Weichteilgewebe zu entfernen, das sich im Gelenkraum befindet.

Chirurgische Shaver sind motorgetriebene Instrumente, die für verschiedene der vorgenannten Anwendungsbereiche geeignet sind, beispielsweise für die (Teil-) Resektion von Meniskusgewebe. Solche chirurgischen Shaver weisen eine um ihre Längsachse drehbare rohrförmige Innenhülse, die mit einem Drehantrieb verbindbar ist, und eine die Innenhülse umgebende, gegenüber dieser drehfeste rohrförmige Außenhülse auf. Die Innenhülse wird von einem Drehantrieb in der Regel oszillierend mit hohen Drehgeschwindigkeiten (bis zu mehreren 1000 Umdrehungen pro Minute) angetrieben. Durch diese (relative) Drehbewegung zwischen der Innen- und der Außenhülse wird ein Schneideffekt erreicht, wenn der Shaver mit seinen Schneidkanten an der Innen- und Außenhülse an den Rand des zu entfernenden Gewebes geführt wird.

Aus US 5 741 287 A ist ein chirurgischer Shaver bekannt, der eine Außenhülse und eine Innenhülse aufweist, die um die Längsachse drehbar innerhalb der drehfesten Außenhülse aufgenommen ist, wobei die Innenhülse mit einem Antriebsmotor verbindbar ist. Die Innenhülse und die Außenhülse weisen konisch zulaufende distale freie Endbereiche auf, wobei angrenzend an die Endbereiche sowohl in der Außenhülse als auch in der Innenhülse jeweils ein ovales Fenster mit Schneidkanten ausgespart ist. Eine Weiterentwicklung eines solchen chirurgischen Shavers mit einer speziellen geometrischen Gestaltung der Fensteröffnungen ist aus EP 2 907 460 B1 bekannt.

Diese bekannten Shaver sind jedoch noch verbesserungswürdig, weil konstruktionsbedingt diese Shaver aufgrund ihrer Bauhöhe und der Anordnung der Schneidkanten z.B. in engen Gelenkspalten das zu entfernende Gewebe zum Schneiden nicht ausreichend erreichen können. In einigen Fällen kann weder ein Shaver noch ein anderes Instrument eingesetzt werden. So ergibt sich häufig eine relativ lange Operationsdauer.

Aus DE10 2019 105 514 A1 ist ein Shaver bekannt. Bei diesem Shaver sind sowohl die Innen- als auch die Außenhülse um eine gemeinsame Längsachse gegenläufig drehbar und die Innenhülse ragt mit ihrem distalen Ende aus dem distalen Ende der Außenhülse hervor und weist im distalen Endbereich schneidende Spitzen auf, mit denen es möglich ist, bei frontaler Zuführung auf ein Gewebe ein Loch in das Gewebe zu schneiden. Mit diesem Shaver ist es jedoch nicht möglich, einen Geweberand zu umgreifen und abzutragen. Aus US 2008/0249553 A1 ist ein Shaver mit den Merkmalen des Oberbegriffs des Patentanspruchs 1 bekannt, der ebenfalls nicht zum Umgreifen und Abtragen eines Geweberandes geeignet ist.

Aufgabe der Erfindung ist es, einen chirurgischen Shaver hinsichtlich seiner Einsatzfähigkeit und Schneidwirkung wesentlich zu verbessern, um eine deutlich kürzere Operationsdauer zu erreichen.

Diese Aufgabe wird mit einem chirurgischen Shaver mit den Merkmalen des Patentanspruchs 1 gelöst. Die weiteren Ausführungsformen werden in den abhängigen Ansprüchen definiert.

Mit einem solchen chirurgischen Shaver lassen sich gegenüber den bekannten Shavern wesentlich bessere Schneidergebnisse bei der Gewebeentfernung erreichen, so dass die Operationsdauer erheblich verkürzt werden kann. Der Shaver wird mit seinem offenen distalen Ende an den Rand des zu entfernenden Gewebes geführt und umgreift den Geweberand, d.h. der Geweberand befindet sich in dieser Situation zwischen dem wenigstens einen Schneidsteg und dem wenigstens einen Gegensteg. Wenn anschließend die Innenhülse in Drehbewegung versetzt wird, schneidet der Shaver nicht wie herkömmliche Shaver mit seiner ersten Schneidkante nur in Längsrichtung, sondern zusätzlich auch mit seiner weiteren nach innen gerichteten Schneidkante in Umfangsrichtung, so dass in wesentlich kürzerer Zeit Gewebe abgetragen werden kann. Außerdem kann der Shaver ohne weitere Maßnahmen auch Gewebe in engen Gelenkspalten entfernen, da er mit seinem distalen freien Endbereich frontal in den Gelenkspalt eingeführt werden kann.

In bevorzugter Ausgestaltung ist vorgesehen, dass die Innenhülse wenigstens einen in Längsrichtung erstreckten, in Längsrichtung vorstehenden zweiten Gegensteg aufweist, der sich nicht bis zum freien Ende des wenigstens einen Schneidsteges erstreckt. Die Außenfläche dieses zweiten Gegensteges ist dabei bevorzugt zu ihrem freien Ende hin leicht konisch zulaufend ausgebildet. Besonders bevorzugt weist die Innenhülse zwei erste und einen zweiten Gegensteg auf.

Nach einer ersten bevorzugten Ausgestaltung ist vorgesehen, dass am stirnseitigen Randbereich des wenigstens einen Schneidsteges eine zweite Schneidkante vorgesehen ist, die eine nach innen gerichtete Komponente in Umfangsrichtung aufweist. Diese zweite Schneidkante ist bei dieser Ausgestaltung dann die weitere Schneidkante. Der erste Gegensteg der Innenhülse ist bei einer Außenhülse mit zweiter Schneidkante nur so lang ausgebildet, dass zwischen der zweiten Schneidkante und dem freien, distalen Ende des ersten Gegensteges ein ausreichend großer ringförmiger Spalt zum Schneiden des Gewebes in Umfangsrichtung verbleibt.

Alternativ oder zusätzlich kann angrenzend an die erste Schneidkante im freien Endbereich des wenigstens einen Schneidsteges eine dritte Schneidkante vorgesehen sein, die eine nach innen gerichtete Komponente in Umfangsrichtung aufweist. Die dritte Schneidkante ist dann die weitere Schneidkante oder es sind zwei weitere Schneidkanten vorgesehen, nämlich die zweite und die dritte.

Weiterhin ist vorteilhaft vorgesehen, dass der wenigstens eine Schneidsteg der Außenhülse an beiden Seiten jeweils eine erste Schneidkante aufweist. Der Schneidsteg ist dann in beiden Drehrichtungen wirksam.

Entsprechend ist bevorzugt vorgesehen, dass der wenigstens eine Schneidsteg der Außenhülse an beiden Seiten jeweils eine dritte Schneidkante aufweist.

In weiterer Ausgestaltung ist vorgesehen, dass die dritte Schneidkante in einer Einbuchtung oder Ausbuchtung des Schneidsteges ausgebildet ist. Wenn der Schneidsteg an beiden Seiten eine dritte Schneidkante aufweist, können beide Schneidkanten in einer Einbuchtung oder Ausbuchtung ausgebildet sein oder alternativ kann eine dritte Schneidkante in einer Einbuchtung und die andere dritte Schneidkante in einer Ausbuchtung ausgebildet sein. Wenn die Außenhülse mehrere Schneidstege aufweist, können deren dritte Schneidkanten ebenfalls in der vorbeschriebenen Weise übereinstimmend oder unterschiedlich ausgebildet sein.

Ferner kann vorteilhaft vorgesehen sein, dass die erste Schneidkante wenigstens zwei Schneidabschnitte aufweist, die gegenüber der Längsachse eine unterschiedliche Neigung aufweisen.

Eine weitere Ausgestaltung zeichnet sich dadurch aus, dass die erste Schneidkante wenigstens bereichsweise bogenförmig ausgebildet ist.

Auch die dritte Schneidkante kann wenigstens bereichsweise bogenförmig ausgebildet sein, bevorzugt dann, wenn sie in einer Ein- oder Ausbuchtung ausgebildet ist.

Auch die Kanten des oder der Gegenstege(s) der Innenhülse können wenigstens bereichsweise als Schneidkanten ausgebildet sein.

Die Erfindung ist nachstehend anhand der Zeichnung beispielhaft näher erläutert. Diese zeigt jeweils in perspektivischer Darstellung in
- Fig. 1: eine vollständige Ansicht eines chirurgischen Shavers,
- Fig. 2: eine Teilansicht eines Shavers in einer ersten Ausführungsform,
- Fig. 3: den Shaver nach Fig. 2 in einer zweiten Position,
- Fig. 4: den Shaver nach Fig. 2 in einer dritten Position,
- Fig. 5: eine Teilansicht der Außenhülse des Shavers nach Fig. 2,
- Fig. 6: die Außenhülse nach Fig. 5 aus einer anderen Perspektive,
- Fig. 7: eine Teilansicht der Innenhülse des Shavers nach Fig. 2,
- Fig. 8: die Innenhülse nach Fig. 7 aus einer zweiten Perspektive,
- Fig. 9: die Innenhülse nach Fig. 7 aus einer dritten Perspektive,
- Fig. 10: eine Teilansicht einer Außenhülse in einer zweiten Ausführungsform,
- Fig. 11: die Außenhülse nach Fig. 10 aus einer zweiten Perspektive,
- Fig. 12: die Außenhülse nach Fig. 10 aus einer dritten Perspektive,
- Fig. 13: eine Teilansicht eines Shavers mit einer Außenhülse nach Fig. 10 und mit einer Innenhülse nach Fig. 7,
- Fig. 14: den Shaver nach Fig. 13 in einer zweiten Position,
- Fig. 15: den Shaver nach Fig. 13 in einer dritten Position,
- Fig. 16: eine Teilansicht einer Außenhülse in einer dritten Ausführungsform,
- Fig. 17: eine Teilansicht einer Außenhülse in einer vierten Ausführungsform,
- Fig. 18: eine Teilansicht einer Außenhülse in einer fünften Ausführungsform,
- Fig. 19: eine Teilansicht einer Außenhülse in einer sechsten Ausführungsform,
- Fig. 20: eine Teilansicht einer Außenhülse in einer siebten Ausführungsform,
- Fig. 21: die Außenhülse nach Fig. 20 aus einer zweiten Perspektive,
- Fig. 22: eine Teilansicht einer Außenhülse in einer achten Ausführungsform,
- Fig. 23: die Außenhülse nach Fig. 22 aus einer zweiten Perspektive,
- Fig. 24: eine Teilansicht einer Außenhülse in einer neunten Ausführungsform und
- Fig. 25: eine Teilansicht einer Außenhülse in einer zehnten Ausführungsform.

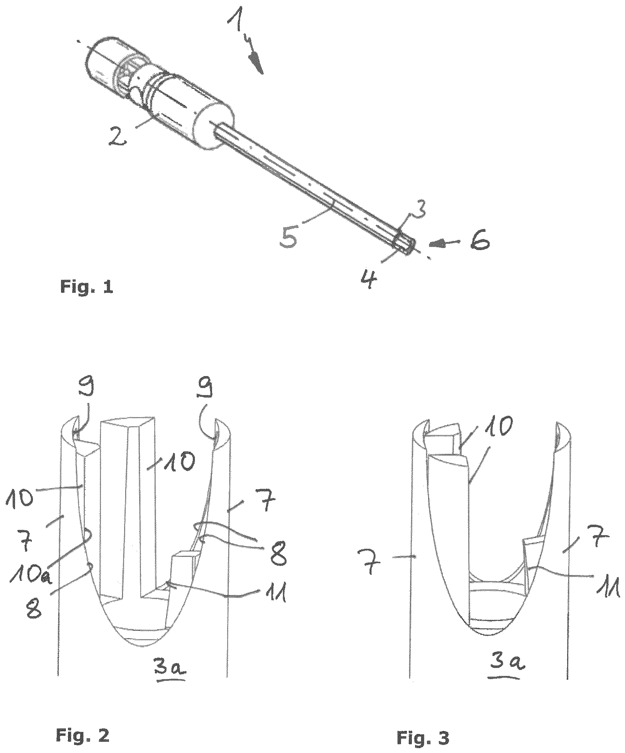

Ein erfindungsgemäßer chirurgischer Shaver ist in Fig. 1 allgemein mit 1 bezeichnet. Dieser Shaver 1 weist am hinteren Ende ein allgemein mit 2 bezeichnetes Kopplungsstück zum Ankuppeln an einen nicht dargestellten Drehantrieb auf. Am Kopplungsstück 2 ist drehfest eine rohrförmige Außenhülse 3 angeordnet. Innerhalb dieser Außenhülse 3 ist eine rohrförmige Innenhülse 4 aufgenommen, welche gegenüber der Außenhülse 3 um eine Längsachse 5 des Shavers 1 drehbar ist. Dazu ist die Innenhülse 4 in üblicher Weise über das Kopplungsstück 2 mit der Drehwelle des Drehantriebes gekoppelt. Der Drehantrieb kann die Innenhülse 4 mit hohen Umdrehungszahlen (einige 1000 Umdrehungen pro Minute) vorzugsweise oszillierend in Drehbewegung versetzen. Der Ringspalt zwischen der Innenhülse 4 und Außenhülse 3 ist so dimensioniert, dass die Innenhülse 4 einerseits weitgehend zentrisch zur Längsachse 5 geführt und andererseits frei drehbar innerhalb der Außenhülse 3 aufgenommen ist.

Erfindungswesentlich ist die Ausgestaltung des Shavers 1 in seinem in Fig. 1 allgemein mit 6 bezeichneten freien distalen Endbereich bzw. die Ausgestaltung der Außenhülse 3 und der Innenhülse 4 im freien distalen Endbereich 6. Diese Gestaltung ist in verschiedenen Ausführungsformen in den Figuren 2 bis 25 dargestellt, wobei sowohl die Außenhülse 3 als auch die Innenhülse 4 jeweils nur im freien Endbereich 6 des Shavers 1 dargestellt sind. Die freien distalen Enden der Innenhülse 4 und der Außenhülse 5 sind bei allen Ausführungsformen in Längsrichtung, also in Richtung der Längsachse 5 offen, so dass der Shaver 1 mit seinem freien Endbereich 6 den Rand eines nicht dargestellten, abzutrennenden Gewebes (zum Beispiel eines Meniskusgewebes) umgreifen kann, wenn die Längsachse 5 des Shavers 1 in etwa mit der Fläche des Gewebes zusammenfällt.

Eine erste Ausführungsform der Außenhülse 3 und der Innenhülse 4 ist in den Figuren 2 bis 9 stark vergrößert dargestellt. Die Außenhülse 3 weist angrenzend an ihren rohrförmigen Bereich 3a im Bereich ihres freien Endes wenigstens einen in Längsrichtung erstreckten, in Längsrichtung vorstehenden Schneidsteg 7 auf, wobei bei allen dargestellten Ausführungsformen zwei Schneidstege 7 vorgesehen sind. Jeder Schneidsteg 7 weist eine erste Schneidkante 8 mit einer Komponente in Längsrichtung auf. Diese erste Schneidkante 8 geht vom Fußbereich 7a des Schneidsteges 7 aus und ist bei der ersten Ausführungsform bogenförmig ausgestaltet. Jeder Schneidsteg 7 weist im freien Endbereich 6 darüber hinaus wenigstens eine weitere Schneidkante mit einer nach innen gerichteten Komponente in Umfangsrichtung auf. Bei der ersten Ausführungsform nach Figuren 2 bis 6 ist diese weitere Schneidkante von einer zweiten Schneidkante 9 am stirnseitigen Randbereich 7b des Schneidsteges 7 gebildet. Beide Schneidstege 7 sind bei der ersten Ausführungsform identisch ausgebildet.

Die Innenhülse 4 weist angrenzend an ihren rohrförmigen Bereich 4a im Bereich ihres freien Endes wenigstens einen in Längsrichtung erstreckten in Längsrichtung vorstehenden ersten Gegensteg 10 auf, der sich im Wesentlichen bis zum freien Ende des wenigstens einen Schneidsteges 7 erstreckt. Wie am besten aus den Figuren 2 bis 4 hervorgeht, ist bei dieser ersten Ausführungsform der erste Gegensteg 10 geringfügig kürzer als der jeweilige Schneidsteg 7, so dass zwischen dem freien Ende des ersten Gegensteges 10 und der zweiten Schneidkante 9 ein ringförmiger Spalt für das abzutrennenden Gewebe verbleibt. Die Längskanten 10a des ersten Gegensteges 10 sind dabei bevorzugt so ausgebildet, dass sie ebenfalls Schneidkanten bilden.

Bei der ersten Ausführungsform weist die Innenhülse 4 zwei identisch gestaltete erste Gegenstege 10 auf. Ferner weist die Innenhülse 4 wenigstens einen in Längsrichtung erstreckten, in Längsrichtung vorstehenden zweiten Gegensteg 11 auf, der sich nicht bis zum freien Ende des wenigstens einen Schneidsteges 7 erstreckt, sondern bevorzugt wesentlich kürzer ausgebildet ist. Dabei ist der zweite Gegensteg 11 vorzugsweise so ausgebildet, dass seine Außenfläche 11a leicht konisch nach innen zulaufend ist, so dass sich der Abstand zwischen dem zweiten Gegensteg 11 und den Schneidstegen 7 in Längsrichtung gesehen zum freien Ende des zweiten Gegensteges 11 hin vergrößert. Die Längskanten 11b des zweiten Gegensteges 10 sind dabei bevorzugt so ausgebildet, dass sie ebenfalls Schneidkanten bilden.

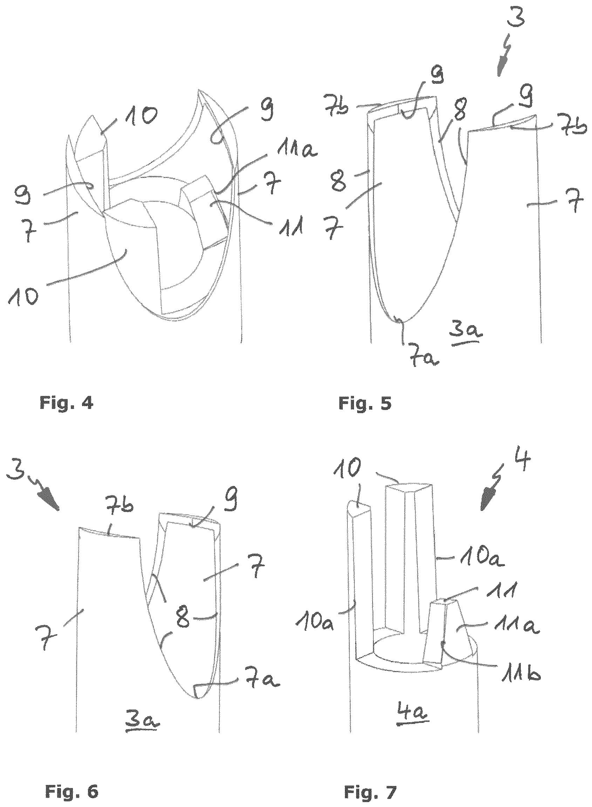

In den Figuren 2 bis 4 sind drei unterschiedliche Drehpositionen zwischen der Außenhülse 3 und der Innenhülse 4 dargestellt. In der Position gemäß Fig. 2 befindet sich ein erster Gegensteg 10 in Anlage (natürlich mit einem gewissen Abstand) an einem Schneidsteg 7, so dass das freie distale Ende des ersten Gegensteges 10 sich im Bereich der zweiten Schneidkante 9 des Schneidsteges 7 befindet und dadurch nicht dargestelltes Gewebe von der zweiten Schneidkante 9 in Umfangsrichtung durchtrennt wird. Außerdem befindet sich eine Längskante 10a des ersten Gegensteges 10 etwa punktförmig in Anlage an der ersten Schneidkante 8. Dadurch wird das nicht dargestellte Gewebe von der ersten Schneidkante 8 im Wesentlichen in Längsrichtung durchtrennt. Die Figuren 3 und 4 zeigen weitere Drehpositionen mit entsprechend geänderten räumlichen Zuordnungen der Schneidstege 7 und der ersten und zweiten Gegenstege 10, 11.

Erkennbar ist, dass durch die Ausgestaltung des Shavers 1, also seines freien Endbereiches 6, abzutrennendes Gewebe nicht nur wie bei bekannten Shavern im Wesentlichen in Längsrichtung durchtrennt wird, sondern zusätzlich auch in Umfangsrichtung. Dadurch lässt sich Gewebe in wesentlich kürzerer Zeit entfernen.

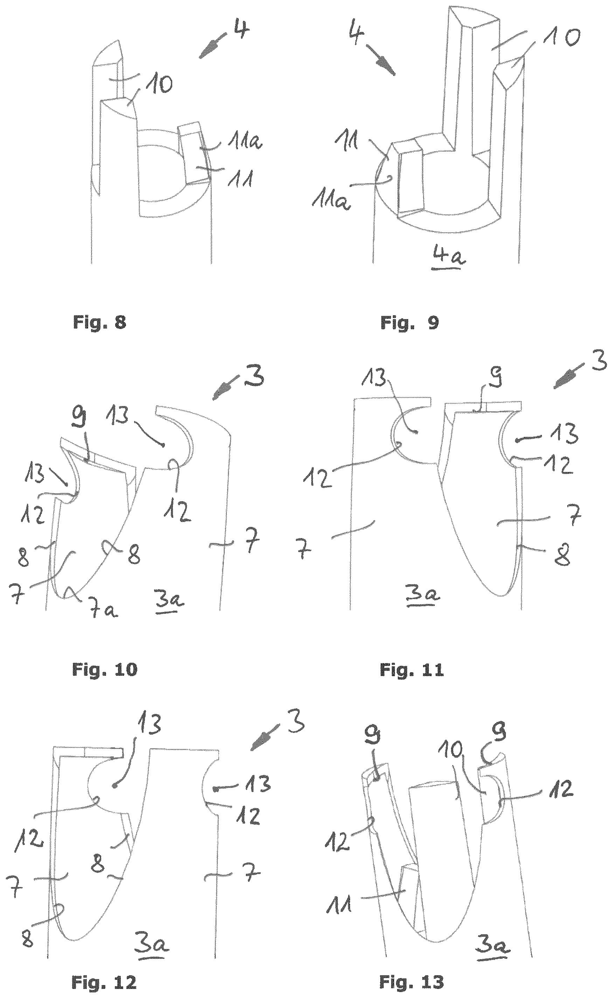

In den Figuren 10 bis 12 ist eine zweite Ausführungsform der Außenhülse 3 dargestellt. Im Unterschied zur ersten Ausführungsform weist jeder Schneidsteg 7 an einer Seite angrenzend an die erste Schneidkante 8 eine weitere Schneidkante, nämlich eine dritte Schneidkante 12, auf, die eine nach innen gerichtete Komponente in Umfangsrichtung aufweist. Diese dritte Schneidkante 12 ist in einer Einbuchtung 13 des Schneidsteges 7 ausgebildet und bogenförmig ausgebildet. Die dritte Schneidkante 12 geht zum freien distalen Ende hin in die zweite Schneidkante 9 über. In Abwandlung dieser Ausführungsform kann die zweite Schneidkante 9 auch entfallen, dann ist die dritte Schneidkante 12 die einzige weitere Schneidkante.

Bei dieser Ausführungsform sind die Einbuchtungen 13 der beiden Schneidstege 7 in Umfangsrichtung gesehen entgegengesetzt zueinander orientiert, so dass je nach Drehrichtung nur eine der beiden dritten Schneidkanten 12 wirksam ist. In weiterer Ausgestaltung können auch an beiden Seiten des jeweiligen Schneidsteges 7 Einbuchtungen 13 mit dritten Schneidkanten 12 vorgesehen sein. Wie dargestellt kann aber die Seite des Schneidsteges 7 ohne Einbuchtung 13 auch so ausgebildet sein wie bei der ersten Ausführungsform.

In den Figuren 13 bis 15 ist die Außenhülse 3 nach der zweiten Ausführungsform zusammen mit einer unveränderten Innenhülse 4 in drei unterschiedlichen Drehpositionen dargestellt.

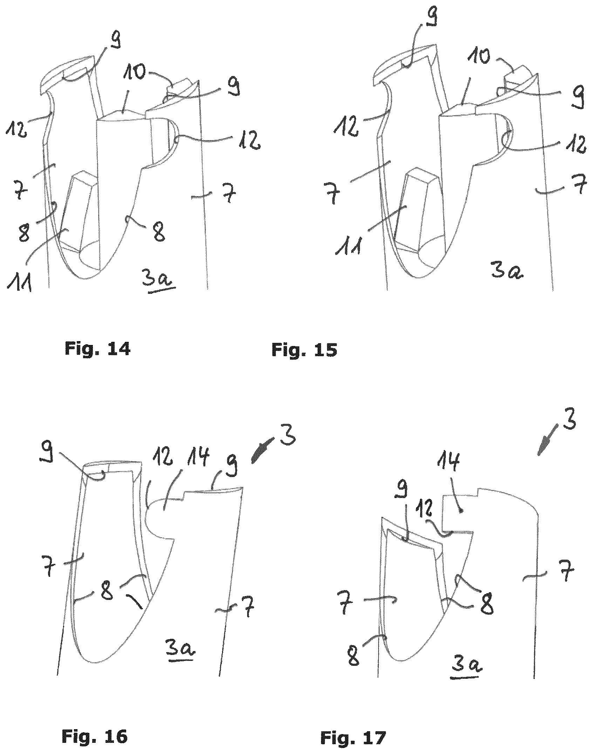

In Fig. 16 ist eine dritte Ausführungsform der Außenhülse 3 dargestellt. Bei dieser Ausführungsform weist die Außenhülse 3 an einem Schneidsteg 7 eine dritte Schneidkante 12 auf, die entlang einer Ausbuchtung 14 bogenförmig ausgebildet ist. Grundsätzlich können auch beide Schneidstege 7 bei dieser dritten Ausführungsform jeweils wenigstens eine Ausbuchtung 14 mit zweiter Schneidkante 12 aufweisen. Es ist auch möglich, dass an einer Seite eines Schneidsteges 7 eine Einbuchtung 13 mit dritter Schneidkante 12 und an der anderen Seite eine Ausbuchtung 14 mit dritter Schneidkante 12 vorgesehen ist. Wenn zwei Schneidstege 7 vorhanden sind, können an diesen in unterschiedlicher Ausrichtung wenigstens eine Einbuchtung 13 und/oder Ausbuchtung 14 vorgesehen sein.

In Fig. 17 ist eine vierte Ausführungsform der Außenhülse 3 dargestellt, die sich von derjenigen nach Fig. 16 nur dadurch unterscheidet, dass die Ausbuchtung 14 und die an dieser ausgebildete zweite Schneidkante 12 nicht bogenförmig, sondern rechteckig ausgebildet ist.

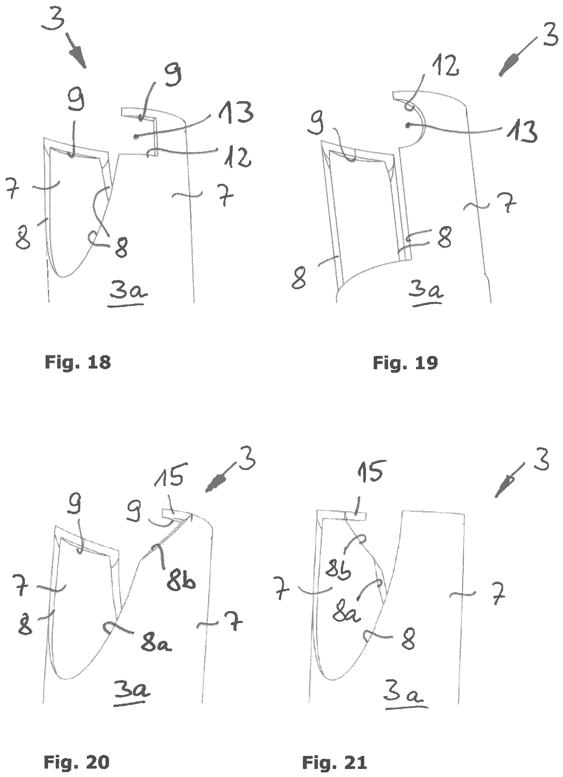

Fig. 18 zeigt eine fünfte Ausführungsform der Außenhülse 3. Bei dieser Ausführungsform ist an einem Schneidsteg 7 eine Einbuchtung 13 mit rechteckiger Form vorgesehen. Die dritte Schneidkante 12 geht dabei in die zweite Schneidkante 9 über. Bei dieser Ausführungsform ist im Unterschied zur zweiten Ausführungsform nur an einem Schneidsteg 7 eine Einbuchtung 13 vorgesehen.

Fig. 19 zeigt eine sechste Ausführungsform der Außenhülse 3. Diese Ausführungsform unterscheidet sich von derjenigen nach Fig. 18 nur dadurch, dass die Einbuchtung 13 und damit der Verlauf der dritten Schneidkante 12 bogenförmig ist.

Die Figuren 20 und 21 zeigen eine Außenhülse 3 in einer siebten Ausführungsform. An einem Schneidsteg 7 weist die erste Schneidkante 8 zwei Schneidabschnitte 8a und 8b auf, die gegenüber der Längsachse 4 eine unterschiedliche Neigung aufweisen. Dabei ist ein erster Schneidabschnitt 8a bogenförmig ausgebildet, während der zweite Schneidabschnitt 8b im Wesentlichen geradlinig ausgebildet ist. Des Weiteren ist bei diesem Schneidsteg 7 vorgesehen, dass sich die zweite Schneidkante 9 in einen ringförmigen Steg 15 fortsetzt.

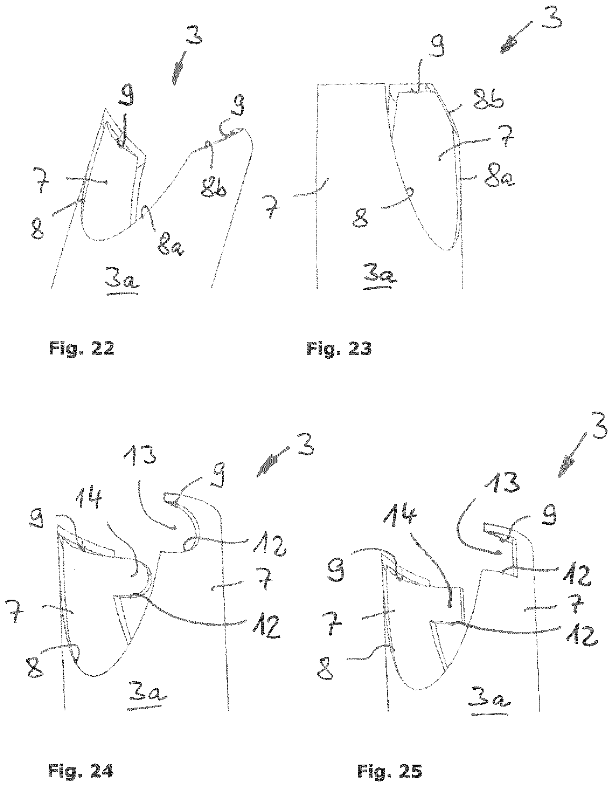

Die Figuren 22 und 23 zeigen eine Außenhülse 3 in einer achten Ausführungsform. Diese achte Ausführungsform der Außenhülse 3 unterscheidet sich von der siebten Ausführungsform nur dadurch, dass der Steg 15 entfällt.

In Fig. 24 ist eine neunte Ausführungsform der Außenhülse 3 dargestellt. Bei dieser Ausführungsform sind zwei Schneidstege 7 vorgesehen, wobei ein Schneidsteg 7 eine bogenförmige Einbuchtung 13 mit dritter Schneidkante 12 und der andere Schneidsteg 7 eine bogenförmige Ausbuchtung 14 mit dritter Schneidkante 12 aufweist. Außerdem sind an beiden Schneidstegen 7 erste und zweite Schneidkanten 8, 9 vorhanden.

Schließlich ist in Fig. 25 eine zehnte Ausführungsform der Außenhülse 3 dargestellt. Diese Ausführungsform unterscheidet sich von der neunten Ausführungsform nur dadurch, dass die Einbuchtung 13 mit dritter Schneidkante 12 an einem Schneidsteg 7 und die Ausbuchtung 14 mit dritter Schneidkante 12 am anderen Schneidsteg 7 nicht bogenförmig, sondern rechteckig ausgebildet sind.

Natürlich ist die Erfindung nicht auf die dargestellten Ausführungsbeispiele beschränkt. Weitere Ausgestaltungen sind möglich, ohne den Grundgedanken zu verlassen. So können die verschiedenen geometrischen Ausgestaltungen der Außenhülse 3 im Rahmen der vorbeschriebenen Ausführungsformen auf beliebige Weise miteinander kombiniert werden. So kann ein Schneidsteg 7 auf einer Seite eine Einbuchtung 13 und auf der anderen Seite eine Ausbuchtung 14 aufweisen. Grundsätzlich kann auch nur ein Schneidsteg 7 vorgesehen sein oder alternativ mehr als zwei. Die Innenhülse 4 kann auch nur einen ersten Gegensteg 10 mit oder ohne zweiten Gegensteg 11 aufweisen.

### Bezugszeichenliste:

- 1: chirurgischer Shaver
- 2: Kopplungsstück
- 3: Außenhülse
- 3a: rohrförmiger Bereich
- 4: Innenhülse
- 4a: rohrförmiger Bereich
- 5: Längsachse
- 6: freier Endbereich
- 7: Schneidsteg
- 7a: Fußbereich
- 7b: Randbereich
- 8: erste Schneidkante
- 8a: erster Schneidabschnitt
- 8b: zweiter Schneidabschnitt
- 9: zweite Schneidkante
- 10: erster Gegensteg
- 10a: Längskante
- 11: zweiter Gegensteg
- 11a: Außenfläche
- 11b: Längskante
- 12: dritte Schneidkante
- 13: Einbuchtung
- 14: Ausbuchtung
- 15: Steg

## Patentansprüche

1. Chirurgischer Shaver mit einer um ihre Längsachse (5) drehbaren rohrförmigen Innenhülse (4), die mit einem Drehantrieb verbindbar ist, und einer die Innenhülse (4) umgebenden, gegenüber dieser drehfesten
rohrförmigen Außenhülse (3), wobei die freien distalen Enden der Innenhülse (4) und der Außenhülse (3) in Längsrichtung offen sind und wobei die Außenhülse (3) im Bereich ihres freien Endes wenigstens einen in Längsrichtung erstreckten, in Längsrichtung vorstehenden Schneidsteg (7) mit wenigstens einer ersten Schneidkante (8) aufweist, die eine Komponente in Längsrichtung aufweist und vom Fußbereich (7a) des Schneidsteges (7) ausgeht, und wobei die Innenhülse (4) im Bereich ihres freien Endes wenigstens einen in Längsrichtung erstreckten, in Längsrichtung vorstehenden ersten Gegensteg (10) aufweist, wobei der wenigstens eine erste Gegensteg (10) der Innenhülse (4) sich im Wesentlichen bis zum freien Ende des wenigstens einen Schneidsteges (7) erstreckt,
**dadurch gekennzeichnet,**
**dass** der wenigstens eine Schneidsteg (7) der Außenhülse (3) wenigstens eine weitere Schneidkante (9,12) im freien Endbereich aufweist, die eine nach innen gerichtete Komponente in Umfangsrichtung aufweist.

2. Shaver nach Anspruch 1,
**dadurch gekennzeichnet,**
**dass** die Innenhülse (4) wenigstens einen in Längsrichtung erstreckten, in Längsrichtung vorstehenden zweiten Gegensteg (11) aufweist, der sich nicht bis zum freien Ende des wenigstens einen Schneidsteges (7) erstreckt.

3. Shaver nach Anspruch 1 oder 2,
**dadurch gekennzeichnet,**
**dass** am stirnseitigen Randbereich (7b) des wenigstens einen Schneidsteges (7) eine zweite Schneidkante (9) vorgesehen ist, die eine nach innen gerichtete Komponente in Umfangsrichtung aufweist.

4. Shaver nach Anspruch 1, 2 oder 3,
**dadurch gekennzeichnet,**
**dass** angrenzend an die erste Schneidkante (8) im freien Endbereich des wenigstens einen Schneidsteges (7) eine dritte Schneidkante (12) vorgesehen ist, die eine nach innen gerichtete Komponente in Umfangsrichtung aufweist.

5. Shaver nach einem oder mehreren der Ansprüche 1 bis 4,
**dadurch gekennzeichnet,**
**dass** der wenigstens eine Schneidsteg (7) der Außenhülse (3) an beiden Seiten jeweils eine erste Schneidkante (8) aufweist.

6. Shaver nach Anspruch 4 oder 5,
**dadurch gekennzeichnet,**
**dass** der wenigstens eine Schneidsteg (7) der Außenhülse (3) an beiden Seiten jeweils eine dritte Schneidkante (12) aufweist.

7. Shaver nach einem oder mehreren der Ansprüche 4 bis 6,
**dadurch gekennzeichnet,**
**dass** die dritte Schneidkante (12) in einer Einbuchtung (13) oder Ausbuchtung (14) des Schneidsteges (7) ausgebildet ist.

8. Shaver nach einem oder mehreren der Ansprüche 1 bis 7,
**dadurch gekennzeichnet,**
**dass** die erste Schneidkante (8) wenigstens zwei Schneidabschnitte (8a,8b) aufweist, die gegenüber der Längsachse eine unterschiedliche Neigung aufweisen.

9. Shaver nach einem oder mehreren der Ansprüche 1 bis 7,
**dadurch gekennzeichnet,**
**dass** die erste Schneidkante (8) wenigstens bereichsweise bogenförmig ausgebildet ist.

10. Shaver nach einem oder mehreren der Ansprüche 1 bis 9,
**dadurch gekennzeichnet,**
**dass** die dritte Schneidkante (12) wenigstens bereichsweise bogenförmig ausgebildet ist.

## Claims

1. Surgical shaver with a tubular inner sleeve (4) which can be rotated about its longitudinal axis (5) and can be connected to a rotation drive, and with a tubular outer sleeve (3) which surrounds the inner sleeve (4) and is rotationally fixed with respect thereto, wherein the free distal ends of the inner sleeve (4) and of the outer sleeve (3) are open in the longitudinal direction, and wherein the outer sleeve (3) has, in the region of its free end, at least one cutting bar (7) extending in the longitudinal direction, projecting in the longitudinal direction and with at least one first cutting edge (8) which has a component in the longitudinal direction and starts from the foot region (7a) of the cutting bar (7), and wherein the inner sleeve (4) has, in the region of its free end, at least one first mating bar (10) extending in the longitudinal direction, projecting in the longitudinal direction and extending substantially up to the free end of the at least one cutting bar (7),
**characterized in that**
the at least one cutting bar (7) of the outer sleeve (3) has at least one further cutting edge (9, 12) in the free end region, which edge has a component that is directed inward in the circumference direction.

2. Shaver according to claim 1,
**characterized in that**
the inner sleeve (4) has at least a second mating bar (11) that extends in the longitudinal direction and projects in the longitudinal direction, which bar does not extend all the way to the free end of the at least one cutting bar (7).

3. Shaver according to claim 1 or 2,
**characterized in that**
a second cutting edge (9) is provided on the end-face edge region (7b) of the at least one cutting bar (7), which edge has a component that is directed inward in the circumference direction.

4. Shaver according to claim 1, 2 or 3,
**characterized in that**
a third cutting edge (12) is provided adjacent to the first cutting edge (8), in the free end region of the at least one cutting bar (7), which edge has a component that is directed inward in the circumference direction.

5. Shaver according to one or more of claims 1 to 4,
**characterized in that**
the at least one cutting bar (7) of the outer sleeve (3) has a first cutting edge (8) on both sides, in each instance.

6. Shaver according to claim 4 or 5,
**characterized in that**
the at least one cutting bar (7) of the outer sleeve (3) has a third cutting edge (12) on both sides, in each instance.

7. Shaver according to one or more of claims 4 to 6,
**characterized in that**
the third cutting edge (12) is formed in an indentation (13) or convexity (14) of the cutting bar (7).

8. Shaver according to one or more of claims 1 to 7,
**characterized in that**
the first cutting edge (8) has at least two cutting sections (8a, 8b) which have a different inclination relative to the longitudinal axis.

9. Shaver according to one or more of claims 1 to 7,
**characterized in that**
the first cutting edge (8) is formed in arc shape, at least in certain regions.

10. Shaver according to one or more of claims 1 to 9,
**characterized in that**
the third cutting edge (12) is formed in arc shape, at least in certain regions.

## Revendications

1. Shaver chirurgical avec une gaine intérieure (4) tubulaire rotative autour de son axe longitudinal (5) et pouvant être connectée à une commande de rotation, et une gaine extérieure (3) tubulaire entourant la gaine intérieure (4) et non rotative par rapport à celle-ci, dans lequel les extrémités distales libres de la gaine intérieure (4) et de la gaine extérieure (3) sont ouvertes dans la direction longitudinale et dans lequel la gaine extérieure (3) présente, dans la zone de son extrémité libre, au moins une nervure de coupe (7) s'étendant dans la direction longitudinale et faisant saillie dans la direction longitudinale, avec au moins une première arête de coupe (8) qui présente un composant dans la direction longitudinale et qui part de la zone de pied (7a) de la nervure de coupe (7), et dans lequel la gaine intérieure (4) présente, dans la zone de son extrémité libre, au moins une première contre-nervure (10) s'étendant dans la direction longitudinale et faisant saillie dans la direction longitudinale, dans lequel la au moins une première contre-nervure (10) de la gaine intérieure (4) s'étend sensiblementjusqu'à l'extrémité libre de la au moins une nervure de coupe (7),
**caractérisé en ce que**
la au moins une nervure de coupe (7) de la gaine extérieure (3) présente au moins une arête de coupe supplémentaire (9, 12) dans la zone de l'extrémité libre, qui présente un composant orienté vers l'intérieur dans la direction circonférentielle.

2. Shaver selon la revendication 1,
**caractérisé en ce que**
la gaine intérieure (4) présente au moins une deuxième contre-nervure (11) s'étendant dans la direction longitudinale et faisant saillie dans la direction longitudinale, qui ne s'étend pas jusqu'à l'extrémité libre de la au moins une nervure de coupe (7).

3. Shaver selon la revendication 1 ou 2,
**caractérisé en ce que**
une deuxième arête de coupe (9) est prévue sur la zone de bord frontale (7b) de la au moins une nervure de coupe (7), qui présente un composant orienté vers l'intérieur dans la direction circonférentielle.

4. Shaver selon la revendication 1, 2 ou 3,
**caractérisé en ce que**
une troisième arête de coupe (12) est prévue dans la zone d'extrémité libre de la au moins une nervure de coupe (7) de manière adjacente à la première arête de coupe (8), qui présente un composant orienté vers l'intérieur dans la direction circonférentielle.

5. Shaver selon une ou plusieurs des revendications 1 à 4,
**caractérisé en ce que**
la au moins une nervure de coupe (7) de la gaine extérieure (3) présente une première arête de coupe (8) respectivement des deux côtés.

6. Shaver selon la revendication 4 ou 5,
**caractérisé en ce que**
la au moins une nervure de coupe (7) de la gaine extérieure (3) présente une troisième arête de coupe (12) respectivement des deux côtés.

7. Shaver selon une ou plusieurs des revendications 4 à 6,
**caractérisé en ce que**
la troisième arête de coupe (12) est conçue dans une échancrure (13) ou une courbure (14) de la nervure de coupe (7).

8. Shaver selon une ou plusieurs des revendications 1 à 7,
**caractérisé en ce que**
la première arête de coupe (8) présente au moins deux sections de coupe (8a, 8b) qui présentent une inclinaison différente par rapport à l'axe longitudinal.

9. Shaver selon une ou plusieurs des revendications 1 à 7,
**caractérisé en ce que**
la première arête de coupe (8) est conçue au moins par zones en forme d'arc.

10. Shaver selon une ou plusieurs des revendications 1 à 9,
**caractérisé en ce que**
la troisième arête de coupe (12) est conçue au moins par zones en forme d'arc.
